# FASCICULE DE BREVET EUROPEEN

(11) **EP 0 566 807 B1**
(45) Date de publication et mention de la délivrance du brevet: **11.06.1997**
(21) Numéro de dépôt: 92440049.2
(22) Date de dépôt: 24.04.1992
(51) Int. Cl.: A61F 2/06

(54) **Endoprothèse vasculaire autoexpansible**
Selbstausspreizbare, vaskuläre Endoprothese
Self expanding vascular endoprosthesis

(43) Date de publication de la demande: 27.10.1993
(73) Titulaire: COGENT, 69400 Villefranche-sur-Saône (FR)
(72) Inventeur: Sgro, Jean-Claude, 21000 Dijon (FR)
(74) Mandataire: Guerre, Dominique

(56) Documents cités:
- EP-A- 0 274 846
- EP-A- 0 364 787
- DE-A- 3 417 738
- DE-U- 8 812 719
- DE-U- 9 010 130
- FR-A- 2 671 280

## Description

La présente invention concerne le domaine médico-chirurgical, notamment de la pathologie artérielle, mais également, d'une façon plus générale, de la pathologie d'autres vaisseaux sanguins, tels que les canaux veineux, ayant pour objet une endoprothèse vasculaire autoexpansible à élasticité permanente et faible raccourcissement comme définie dans le préambule de la revendication 1. Une telle endoprothèse, même si elle est utilisée notamment comme filtre sanguin, est connue du document DE-A-3 417 738.

En l'état actuel de la technique, il existe déjà des prothèses artérielles endoluminales, désignées dans la littérature médicale internationale par le terme anglais "stent".

Ces prothèses sont de deux types :
- les unes en matériel métallique non élastique, expansible de l'intérieur vers l'extérieur sous l'effet d'un ballon gonflable.
- les autres en matériel métallique élastique (voir par exemple DE-U-8 812 719) et/ou constituées de mailles métalliques ; elles sont introduites tendues dans la lumière du vaisseau et leur diamètre augmente lorsque l'extension longitudinale est relâchée.

Ces prothèses présentent divers inconvénients :
- celles du premier type nécessitent d'être soigneusement ajustées sur un ballon car une fois expansées elles ne peuvent plus être retirées.
- celles du deuxième type n'ont pas cet inconvénient, mais la structure de leurs mailles nécessite une extension longitudinale importante pour en réduire le diamètre, condition indispensable pour pouvoir les introduire dans un vaisseau ; une fois introduites leur rétraction à l'intérieur du vaisseau est également très importante, d'où des difficultés d'ajustement et des frottements sur les parois dudit vaisseau sur une longueur de plusieurs centimètres.

Pour éviter la nécessité d'une extension trop importante et les frottements sur les parois, l'endoprothèse selon l'invention est construite selon une géométrie particulière qui en fonde l'originalité.

L'endoprothèse selon l'invention est définie dans la revendication 1 et se caractérise essentiellement en ce qu'elle est globalement cylindrique et constituée de l'assemblage d'axes longitudinaux en nombre pair, faits de matériaux solides et élastiques, auxquels sont solidarisés, disposés transversalement selon un angle déterminé, des brins également élastiques formant entre deux axes voisins des échelles régulières, les différentes échelles réunies longitudinalement formant un cylindre largement ajouré.

L'endoprothèse selon l'invention est constituée de l'assemblage d'éléments unitaires comprenant trois axes : à partir des deux axes extérieurs, des brins élastiques transversaux rejoignent l'axe intermédiaire sous un angle convergent, lesdits brins transversaux étant en forme de "V" ouvert formant avec l'axe intermédiaire une structure en "arêtes de poisson".

Lorsqu'on exerce sur les axes externes et l'axe intermédiaire des tractions longitudinales en sens inverse, les "v" se ferment, rapprochant les axes externes de l'axe intermédiaire. Le déplacement longitudinal des axes est très faible pour obtenir la fermeture des "V" et le rapprochement des axes.

Les tractions exercées sur la prothèse entière en tirant en sens inverse les axes longitudinaux voisins provoquent une diminution du diamètre du cylindre en rapprochant lesdits axes. On peut ainsi passer d'un tube cylindrique largement ajouré d'un rayon donné à un tube d'un rayon moindre par une extension de faible importance et par un faible déplacement longitudinal des axes.

La structure des brins en "V" étant élastique, le tube reprend sa forme initiale lorsque la traction est relâchée.

Le diamètre de la prothèse selon l'invention est fonction du canal anatomique à appareiller, pouvant varier de quelques millimètres à quelques centimètres, tandis que sa longueur est également adaptée selon les besoins.

Même si la présente invention comprend seulement la prothèse qui vient d'être décrite, la description se réfère pour des raisons éxplicatives également à son système d'application, composé de deux cylindres concentriques de faibles diamètres, l'extrémité distale de l'endoprothèse étant fixée à l'extrémité du cylindre de plus petit diamètre et son extrémité proximale à l'extrémité du cylindre de plus grand diamètre.

En tirant sur le cylindre de plus grand diamètre on étend l'endoprothèse pour en réduire le diamètre. Ainsi tendue elle peut être amenée à l'endroit désiré ; elle est alors détendue pour repousser les parois de l'artère. Elle peut être tendue à nouveau pour la déplacer, si nécessaire, tant qu'elle n'est pas désolidarisée du système d'application. Pendant l'introduction et le déplacement, l'ensemble peut être recouvert d'un troisième cylindre qui protège la prothèse et évite tout accrochage sur les parois des vaisseaux.

L'endoprothèse selon l'invention est réalisée en un matériau susceptible de conserver une élasticité permanente de manière à pouvoir écarter les parois, par exemple d'une artère, et se maintenir en place malgré l'écoulement du flux sanguin et d'éventuels élargissements du diamètre des vaisseaux du fait de leur propre élasticité et des variations alternatives de la pression.

Le matériau employé peut être soit un métal élastique, soit un alliage, soit une matière plastique pouvant conserver son élasticité dans le temps.

L'endoprothèse selon l'invention peut d'autre part être recouverte soit d'un enduit biologique, soit d'une substance protectrice offrant peu d'adhérence au sang devant s'écouler à travers elle.

La prothèse selon l'invention, du fait de sa géométrie, présente l'avantage d'être élastique, autoexpansible à faible raccourcissement, et repliable tant qu'elle n'est pas désolidarisée de son système d'application.

La présente invention sera mieux comprise à la lecture de la description qui suit de certains de ses modes de réalisation, illustrés par le dessin annexé, étant entendu que cette description ne présente aucun caractère limitatif vis à vis de l'invention.

Dans le dessin annexé :
- la figure 1 représente une vue partielle d'un élément unitaire de l'endoprothèse selon l'invention.
- la figure 2 représente le même élément après extension.
- les figures 3, 4 et 5 représentent des endoprothèses obtenues par la juxtaposition longitudinale de respectivement 2, 3 ou 4 éléments unitaires de la figure 1.
- la figure 6 représente en vue en perspective avec arraché partiel un système d'application de la prothèse selon l'invention.
- la figure 7 représente en vue en perspective avec arraché partiel le système d'application de la figure 6 sur lequel est fixée l'endoprothèse.
- la figure 8 représente en vue en perspective avec arraché partiel le même système d'application sur lequel est tendue l'endoprothèse.
- les figures 9a, 9b et 9c représentent en coupes transversales, avant, pendant et après l'extension, la position des principaux axes autour de l'axe entral du système d'application ; la figure 9c montrant la position des axes lorsque là prothèse a été placée dans une artère.
- la figure 10 représente l'anneau formé par les brins transversaux de l'élément unitaire de la figure 1.
- la figure 11 représente une vue détaillée de l'assemblage des brins et des axes de l'endoprothèse selon l'invention.

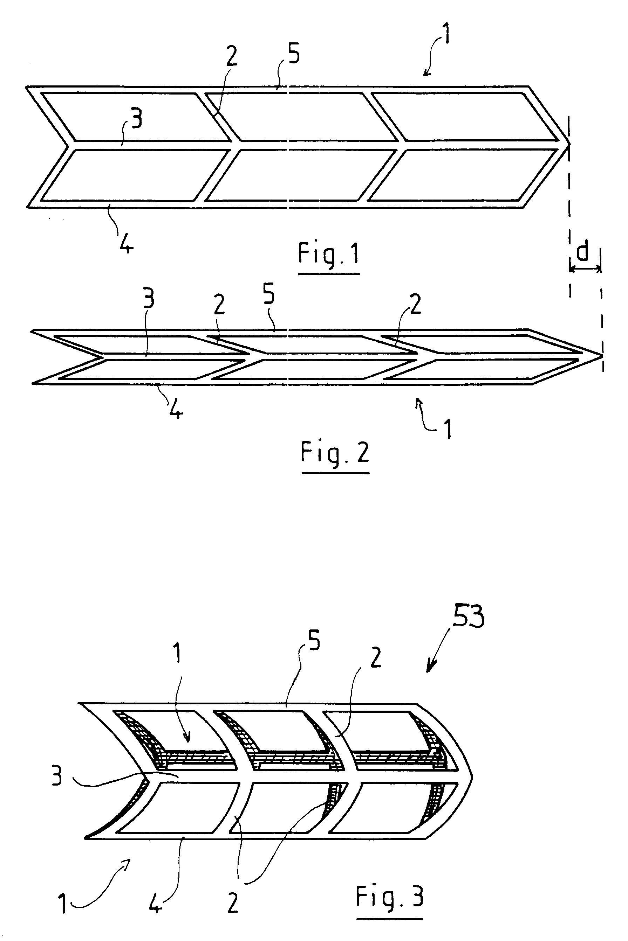

Si on se réfère d'abord aux figure 1 et 2, on voit qu'un élément unitaire 1 de l'endoprothèse selon l'invention est constitué de deux axes externes 4 et 5 solidarisés à un axe intermédiaire 3 au moyen de brins 2 disposés en V dans le même sens, en arête de poisson ou en chevron.

Pour tendre cet élément unitaire 1, il faut solliciter les axes longitudinaux voisins en sens contraire, c'est à dire les axes 4 et 5 en sens contraire de l'axe 3. Les "V" étant tous disposés dans le même sens, l'allongement est minimum lorsqu'on étire l'élément 1, contrairement aux prothèses maillées existantes, dont les mailles forment des losanges qui s'allongent à la traction, chaque allongement s'ajoutant à celui du losange voisin en sorte que l'on finit par obtenir un allongement total très important.

On voit sur la figure 2 l'élément 1 aplati sous l'effet des tractions en sens inverse des axes externes 4 et 5 et de l'axe intermédiaire 3.

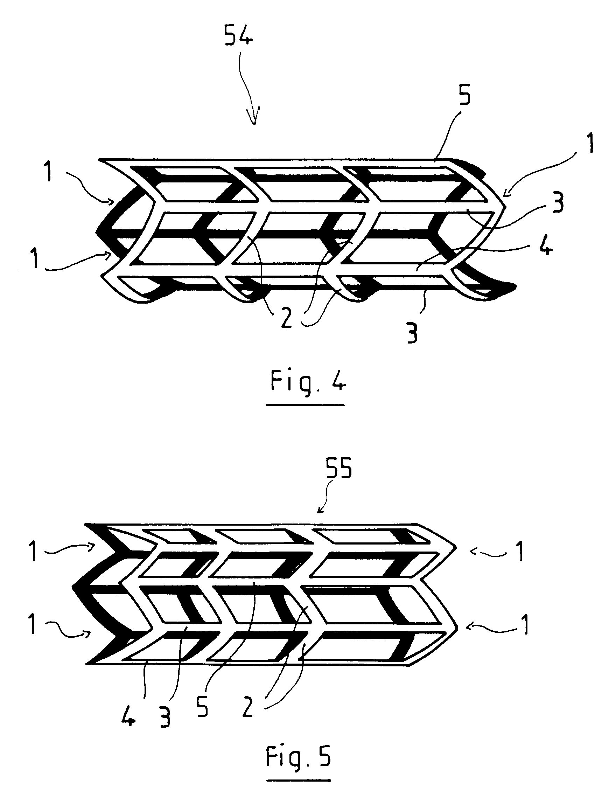

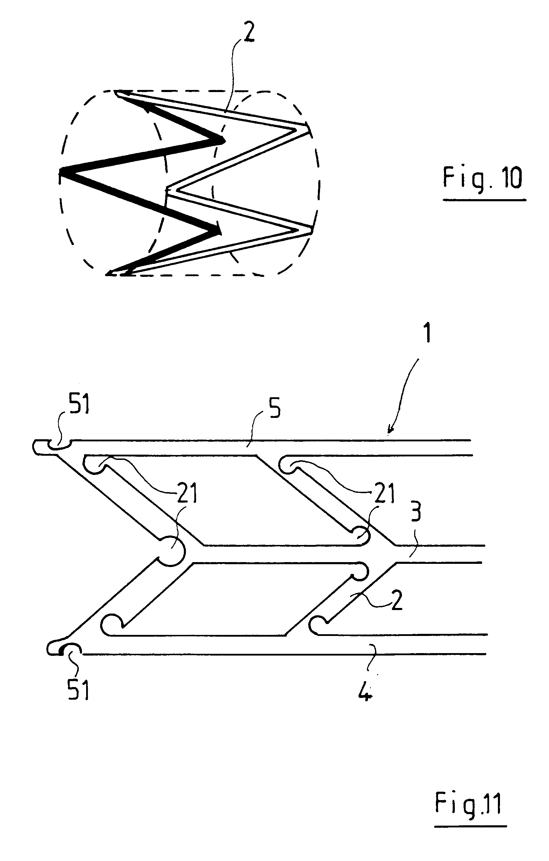

Si on se réfère aux figures 3, 4 et 5, on voit que l'assemblage par juxtaposition longitudinale de 2, 3 ou 4 éléments unitaires 1 permet d'obtenir des cylindres 53, 54, 55 élastiques, dont les tractions des axes voisins en sens inverse permet de réduire le diamètre. Le relâchement de la traction dans un vaisseau de calibre adapté permet à l'endoprothèse de rester fermement appliquée à ses parois (voir figure 9c) du fait de l'élasticité de ses brins. La forme des brins en "V" permet une élasticité circulaire régulière du fait de la force d'écartement qu'ils appliquent à leur base, l'ensemble des "V" contigus formant un anneau régulier dont la lumière interne est circulaire et régulière (voir figure 10).

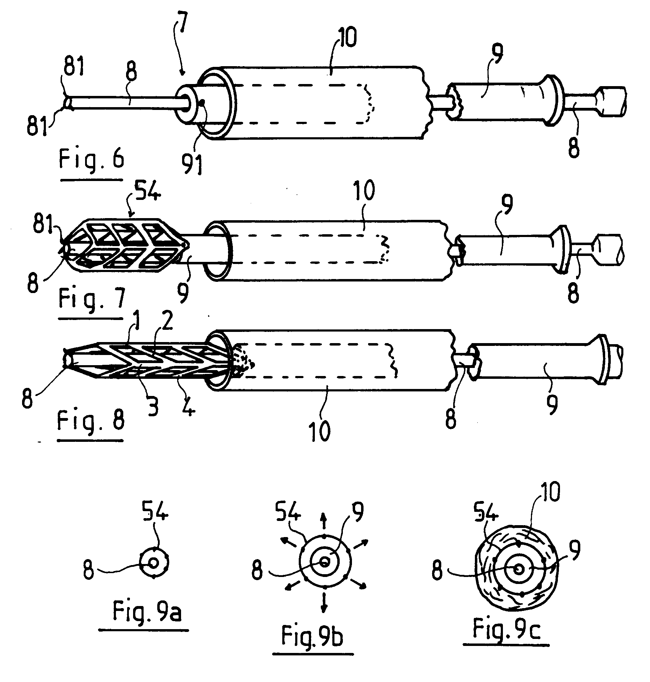

Pour son introduction, par exemple dans un vaisseau sanguin dont on veut maintenir les parois écartées, l'endoprothèse est placée sur le système d'application 7 représenté aux figures 6 à 8, qui permet de l'amener à l'endroit désiré.

Le dispositif d'application 7 est constitué de deux cylindres concentriques 8 et 9 coulissant l'un sur l'autre. La partie distale du cylindre interne 8 est munie d'ergots 81 sur lesquels est fixée une extrémité de l'endoprothèse 54, dont l'autre extrémité est fixée sur le cylindre externe 9 muni à son extrémité distale du même nombre d'ergots 91.

En faisant coulisser le cylindre externe 9 sur le cylindre interne 8 on étire l'endoprothèse 54 en réduisant son diamètre. En relâchant la tension l'augmentation de son diamètre lui permet de se fixer sur la paroi du vaisseau 10 (voir figure 9), qu'elle maintient ouvert.

La fixation de l'endoprothèse 54 sur le dispositif d'application 7 s'effectue au niveau d'orifices disposés à ses extrémités et constitués par le rapprochement, lors de l'assemblage des éléments unitaires 1, d'encoches 51 ménagées aux extrémités desdits éléments unitaires 1, ainsi qu'on peut l'observer sur la figure 11.

Sur cette même figure 11 on voit que les brins transversaux 2 peuvent être munis d'encoches 21 au voisinage de leurs extrémités afin de faciliter la fermeture des angles qu'ils forment avec les axes 3, 4 et 5 lors de la mise en place de l'endoprothèse.

## Revendications

1. Endoprothèse autoexpansible à faible raccourcissement destinée à maintenir écartées les parois d'un vaisseau sanguin, constituée d'éléments unitaires (1) assemblés par juxtaposition longitudinale de manière à former un cylindre ajouré, chaque élément unitaire comprenant trois axes longitudinaux (3, 4, 5), souples et élastiques, parallèles et externes, dont un (3) est positionné de manière intermédiaire, entre les deux autres axes externes (4, 5), et des brins transversaux (2) également souples et élastiques, **caractérisée en ce que** les brins (2) relient les deux axes externes (4, 5) à l'axe intermédiaire externe (3), et sont disposés dans le même sens de manière à former entre eux des "V" ouverts pour constituer avec l'axe intermédiaire une structure en arête de poisson, et en ce qu'un ensemble de "V" contigus forme un anneau régulier dont la lumière interne est circulaire et régulière.

2. Endoprothèse selon la revendication 1, caractérisée en ce qu'elle est constituée de trois ou quatre éléments unitaires.

3. Endoprothèse selon la revendication 1, caractérisée en ce qu'elle est réalisée en un matériau choisi dans le groupe consistant en un métal, un alliage métallique, et une matière plastique.

4. Endoprothèse selon la revendication 1, caractérisée en ce qu'elle est recouverte d'un enduit biologique ou d'une substance protectrice peu adhérente à tout liquide qui s'y écoule.

5. Endoprothèse selon la revendication 1, caractérisée en ce que les axes longitudinaux (3, 4, 5) et/ou les brins transversaux (2) comportent des encoches (51).

## Claims

1. Self-expanding endoprosthesis with low shortening intended to keep the walls of a blood vessel spread apart, consisting of unitary elements (1) assembled by longitudinal juxtaposition in such a way as to form an openworked cylinder, each unitary element comprising three flexible and elastic longitudinal shafts (3, 4, 5) which are parallel and external, of which one (3) is positioned in intermediate manner between the other two outer shafts (4, 5), and transverse ribs (2) which are also flexible and elastic, characterized in that the ribs (2) connect the two outer shafts (4, 5) to the outer intermediate shaft (3) and are arranged in the same direction, in such a way as to form between each other open "V"s in order to constitute, with the intermediate shaft, a fishbone structure, and in that an assembly of contiguous "V"s forms a regular ring whose internal lumen is circular and regular.

2. Endoprosthesis according to Claim 1, characterized in that it is made up of three or four unitary elements.

3. Endoprosthesis according to Claim 1, characterized in that it is made of a material chosen from the group consisting of a metal, a metal alloy, and a plastic material.

4. Endoprosthesis according to Claim 1, characterized in that it is covered by a biological coating or a protective substance with little adherence to any liquid flowing therein.

5. Endoprosthesis according to Claim 1, characterized in that the longitudinal shafts (3, 4, 5) and/or the transverse ribs (2) include notches (51).

## Patentansprüche

1. Endoprothese, die bei geringfügiger Verkürzung selbstausdehnbar ist, und die dazu bestimmt ist, die Wände eines Blutgefäßes gespreizt zu halten, und die aus Einheitselementen (1) besteht, die in Längsrichtung nebeneinander angeordnet und derart zusammengefügt sind, daß sie einen durchbrochenen Zylinder bilden, wobei jedes Einheitselement drei parallele äußere, biegsame und elastische längsverlaufende Achsen (3, 4, 5) aufweist, von denen eine (3) zwischen den beiden anderen äußeren Achsen (4, 5) liegend angeordnet ist, und wobei jedes Einheitselement ebenfalls biegsame und elastische querverlaufende Streben (2) aufweist, dadurch gekennzeichnet, daß die Streben (2) die beiden äußeren Achsen (4, 5) mit der zwischenliegenden äußeren Achse (3) verbinden und in der gleichen Richtung derart angeordnet sind, daß sie miteinander offene "V"'s bilden, um mit der zwischenliegenden Achse eine Struktur in Form eines Fischgrätenmusters zu bilden, und daß eine Anordnung von aneinanderstoßenden "V"'s einen gleichmäßigen Ring bildet, dessen lichter Innenraum kreisförmig und gleichmäßig ist.

2. Endoprothese nach Anspruch 1, dadurch gekennzeichnet, daß sie aus drei oder vier Einheitselementen besteht.

3. Endoprothese nach Anspruch 1, dadurch gekennzeichnet, daß sie aus einem Material gefertigt ist, das ausgewählt ist aus der Gruppe bestehend aus Metallen, Metallegierungen und Kunststoffen.

4. Endoprothese nach Anspruch 1, dadurch gekennzeichnet, daß sie mit einem biologischen Belag oder mit einer Schutzsubstanz bedeckt ist, die gegenüber jeder durch sie fließenden Flüssigkeit wenig adhäsiv ist.

5. Endoprothese nach Anspruch 1, dadurch gekennzeichnet, daß die längsverlaufenden Achsen (3, 4, 5) und/oder die querverlaufenden Streben (2) Einkerbungen (51) aufweisen.
